# EUROPÄISCHE PATENTSCHRIFT

(11) **EP 2 536 439 B1**
(45) Veröffentlichungstag und Bekanntmachung des Hinweises auf die Patenterteilung: **02.11.2016**
(21) Anmeldenummer: 11706501.1
(22) Anmeldetag: 17.02.2011
(51) Int. Cl.: A61L 2/07, A61L 2/24

(54) **GERÄT ZUM DESINIFIZIEREN, STERILISIEREN UND/ODER PFLEGEN VON ÄRZTLICHEN, INSBESONDERE ZAHNÄRZTLICHEN INSTRUMENTEN**
DEVICE FOR DISINFECTING, STERILIZING AND/OR MAINTAINING MEDICAL, ESPECIALLY DENTAL INSTRUMENTS
APPAREIL DE DÉSINFECTION, DE STÉRILISATION ET/OU D'ENTRETIEN D'INSTRUMENTS MÉDICAUX, NOTAMMENT DENTAIRES

(30) Priorität: 17.02.2010 DE 102010002038
(43) Veröffentlichungstag der Anmeldung: 26.12.2012
(73) Patentinhaber: Kaltenbach & Voigt GmbH, 88400 Biberach (DE)
(72) Erfinder: HECKENBERGER, Hans, 88433 Aßmannshardt (DE); WIEK, Hans-Dieter, 88454 Hochdorf 2 (DE); GUGEL, Bernd, 89079 Ulm (DE)
(74) Vertreter: Thun, Clemens
(86) Internationale Anmeldenummer: PCT/EP2011/052326
(87) Internationale Veröffentlichungsnummer: WO 2011/101396

(56) Entgegenhaltungen:
- EP-A1- 0 638 297
- EP-A1- 1 749 502
- EP-A2- 0 890 337
- WO-A2-2008/138645
- DE-A1- 4 323 815
- DE-A1- 19 721 538
- DE-A1-102008 020 586
- US-A- 5 197 499

## Beschreibung

Die vorliegende Erfindung betrifft ein Gerät, welches zum Desinfizieren, Sterilisieren und/oder Pflegen von ärztlichen Instrumenten vorgesehen ist. Insbesondere sollen mit dem Gerät zahnärztliche Instrumente aufbereitet werden.

Bei ärztlichen oder zahnärztlichen Handstücken handelt es sich um rohrförmige Teile, die der Arzt während der Behandlung als Griffhülse ergreift. Ein üblicherweise in der zahnmedizinischen Praxis verwendetes Handstück ist ein sogenanntes Bohrhandstück, das an seinem vorderen Ende ein Behandlungswerkzeug, insbesondere einen Bohrer trägt und mit seinem hinteren Ende mittels einer Kupplung mit einem Versorgungsschlauch gekoppelt ist. Durch das Handstück erstrecken sich Versorgungsleitungen für Energie zum Antrieb des Behandlungsinstruments sowie Fluidleitungen für Behandlungsmedien, beispielsweise Luft und/oder Wasser. Unterschieden wird oftmals zwischen sogenannten Turbinen-Handstücken, bei denen zur Versorgung einer im vorderen Endbereich angeordneten Turbine Druckluft vorgesehen ist, und sogenannten Motor-Handstücken, welche als Antriebseinheit einen Elektromotor aufweisen.

Zur Aufrechterhaltung der Funktion der Handstücke bedarf es von Zeit zu Zeit einer Pflege, insbesondere der drehbar gelagerten Antriebselemente. Ferner führen die in der zahnärztlichen Praxis immer weiter ansteigenden Hygieneanforderungen dazu, dass eine Aufbereitung von Handstücken in regelmäßigen zeitlichen Abständen zu erfolgen hat. Die erfolgreiche Aufbereitung und Einhaltung der entsprechenden Vorgaben muss hierbei durch den Zahnarzt lückenlos dokumentiert werden, was einen nicht unerheblichen personellen und organisatorischen Aufwand nach sich zieht.

Eine manuelle Wiederaufbereitung zahnärztlicher Handstücke erfolgte bislang dadurch, dass die Instrumente nach Gebrauch am Patienten zunächst sprühdesinfiziert und äußerlich abgewaschen wurden. Eine Innenreinigung der Instrumente wurde hingegen in der Regel nicht durchgeführt. Zwischenzeitlich existieren auf dem Markt allerdings Reinigungs- und Desinfektions-Geräte, in denen die Instrumente aufbereitet werden, bevor sie einer Ölpflege unterzogen werden. Die maschinelle Aufbereitung bringt deutliche Vorteile gegenüber einem manuellen Pflegen der Instrumente mit sich, da nur ein maschinelles Verfahren eine sichere und reproduzierbare Reinigung und Pflege ermöglicht.

Die bislang bekannten Geräte können allerdings in der Regel lediglich für einzelne Aufbereitungsschritte benutzt werden, sodass jeweils separat eine Reinigung, eine Pflege und eine Sterilisation durchgeführt werden muss. Die Gesamtheit der hierfür erforderlichen Geräte nimmt einen relativ großen Platz in Anspruch, wobei für jedes der Geräte jeweils elektrische, pneumatische und fluidische Anschlüsse erforderlich sind. Die Realisierung einer vollständigen maschinellen Aufbereitung zahnärztlicher Instrumente mittels einzelner Geräte ist dementsprechend sehr umständlich und mit einem hohen Kostenaufwand verbunden.

Ein weiterer Nachteil besteht darin, dass die einzelnen Geräte in der Regel nicht untereinander vernetzt sind, weshalb ein Datenaustausch zwischen den Geräten nicht erfolgen kann. Dies führt wiederum zu einem Mehraufwand des Bedienpersonals, da keine durchgängig automatische Dokumentation der Instrumentenaufbereitung erstellbar ist. Ferner müssen in Zwischenschritten die Instrumente von Gerät zu Gerät manuell weiterbefördert werden, was mit einem intensiven Personaleinsatz und einem großen Zeitbedarf verbunden ist.

Aus der DE 43 23 815 A1 ist eine Vorrichtung zur hygienischen Aufbereitung von zahnmedizinischen Instrumenten bekannt, die eine Kammer zur Aufnahme der Instrumente aufweist, sowie Zuleitungen für Treibluft, Schmieröl, Luft und Wasser. Zur Aufbereitung wird u. a. eine Dampfdesinfektion durchgeführt.

Aus der DE 10 2008 020 586 A1 ist ein Gerät zum Desinfizieren, Sterilisieren und/oder Pflegen von zahnärztlichen Instrumenten bekannt, bei der zur Aufbereitung heißer Dampf in eine Spülkammer geleitet wird. Aus der EP 1 749 502 A1 ist ein weiteres Pflege- oder Reinigungsgerät für medizinische Instrumente bekannt.

Der vorliegenden Erfindung liegt deshalb die Aufgabe zugrunde, ein neuartiges Gerät zum Desinfizieren, Sterilisieren und/oder Pflegen bzw. allgemein zum Aufbereiten von ärztlichen, insbesondere zahnärztlichen Instrumenten anzugeben, mit dem die oben genannten Nachteile vermieden werden.

Die Aufgabe wird durch ein Gerät, welches die Merkmale der unabhängigen Ansprüche aufweist, gelöst. Vorteilhafte Weiterbildungen der Erfindung sind Gegenstand der abhängigen Ansprüche.

Erfindungsgemäß wird dementsprechend ein Gerät zum Desinfizieren, Sterilisieren und/oder Pflegen von ärztlichen, insbesondere zahnärztlichen Instrumenten vorgeschlagen, welches eine Pflege-und Reinigungskammer zur Aufnahme von aufzubereitenden Instrumenten sowie Mittel zum Zuführen unterschiedlicher Reinigungs-oder Pflegemedien aufweist, um die Instrumente in aufeinanderfolgenden Schritten zu reinigen, zu desinfizieren bzw. sterilisieren und/oder zu pflegen. Dabei ist erfindungsgemäß vorgesehen, dass die Aufbereitungszyklen für die verschiedenen Instrumente zeitlich versetzt starten. Das Gerät ist dabei dazu ausgebildet, die Aufbereitungszyklen zeitlich überlagert ablaufen zu lassen.

Die erfindungsgemäße Vorgehensweise hat zur Folge, dass die zum Aufbereiten der Instrumente benötigten Medien nicht zeitgleich mehreren Instrumenten zugeführt werden müssen. Dies erlaubt es, die Medienmenge jeweils exakt zu dosieren. Gleichzeitig kann jedoch der zeitliche Ablauf der Aufbereitung aller Instrumente gestrafft werden, da die Aufbereitungszyklen zeitlich überlagert sind. Letztendlich wird hierdurch ein Gerät geschaffen, mit dem sämtliche zum Aufbereiten eines Instruments erforderlichen Schritte durchgeführt werden können, wobei gleichzeitig die Qualität der Aufbereitung aufgrund der Möglichkeit der exakten Mediendosierung sehr hoch ist und andererseits für die Aufbereitung mehrerer Instrumente verhältnismäßig wenig Zeit in Anspruch genommen wird.

Gemäß einer ersten bevorzugten Ausführungsform der vorliegenden Erfindung erfolgt dabei eine Reinigung und Desinfektion der Instrumente mittels Heißdampf. In diesem Fall ist vorgesehen, dass ein Aufbereitungszyklus für ein Instrument jeweils aus sechs aufeinanderfolgenden Schritten besteht, nämlich Innenreinigung mittels Heißdampf, Außenreinigung mittels Heißdampf, Desinfektion mittels Heißdampf, Ölpflege mittels ölhaltiger Druckluft, Durchblasen mittels Druckluft und weitere Außenreinigung mittels Heißdampf. Ein Aufbereitungszyklus für ein Instrument wird vorzugsweise dann gestartet, wenn das vorherige Instrument mittels ölhaltiger Druckluft gepflegt wird.

Bei einer zweiten bevorzugten Ausführungsform erfolgt eine Desinfektion der Instrumente durch die Zuführung und das Einwirkenlassen eines Desinfektionsmittels, insbesondere eines Sprays, wobei die Einwirkzeit für nachfolgende Instrumente zunimmt. Ein Aufbereitungszyklus für ein Instrument besteht in diesem Fall vorzugsweise aus folgenden Schritten: Innenreinigung durch Zuführen und Einwirkenlassen eines Reinigungsmittels, Desinfektion durch Zuführen und Einwirkenlassen eines Desinfektionsmittels, Ölpflege durch Zuführen eines Ölpflegemittels und Durchblasen des Instruments.

Bei einer dritten bevorzugten Ausführungsform erfolgt eine Innenreinigung der Instrumente durch die Zuführung und das Einwirkenlassen eines Reinigungsmittels, insbesondere eines Reinigungssprays, wobei nunmehr die Einwirkzeit für die nachfolgenden Instrumente zunimmt. Ein Aufbereitungszyklus für ein Instrument besteht in diesem Fall vorzugsweise aus folgenden Schritten: Innenreinigung durch Zuführen und Einwirkenlassen eines Reinigungsmittels, Ölpflege durch Zuführen eines Ölpflegemittels und Durchblasen des Instruments mittels Druckluft. Bei der soeben beschriebenen Variante sowie bei der zuvor genannten Variante wird vorzugsweise ein Aufbereitungszyklus für ein Instrument dann gestartet, wenn die Einwirkzeit für das Reinigungsmittel für das vorherige Instrument beginnt.

Gemäß einer anderen vorteilhaften Weiterbildung der Erfindung kann ferner vorgesehen sein, dass vor einem Zuführen der Reinigungs- oder Pflegemedien ein Durchspülen der Instrumente mit kaltem Wasser unter hohem pulsierendem Druck erfolgt. Es hat sich gezeigt, dass durch diese Maßnahme, die auch unabhängig von den zuvor beschriebenen Erfindungsgedanken zum Einsatz kommen kann, ein besonders effektives Reinigen der Instrumente ermöglicht wird.

Letztendlich wird also ein Gerät vorgeschlagen, welches in vollautomatischer, zuverlässiger und reproduzierbarer Weise ein Aufbereiten insbesondere zahnärztlicher Instrumente ermöglicht.

Nachfolgend soll die Erfindung anhand der beiliegenden Zeichnung näher erläutert werden. Es zeigen:
- Figur 1: schematisch ein erstes Ausführungsbeispiel eines erfindungsgemäßen Geräts zum Desinfizieren, Sterilisieren und/oder Pflegen von zahnärztlichen Instrumenten;
- Figur 2: den zeitlichen Ablauf der Wiederaufbereitung von vier Instrumenten bei dem Ausführungsbeispiel von Fig. 1;
- Figur 3: ein zweite Ausführungsbeispiel eines erfindungsgemäßen Geräts zum Desinfizieren, Sterilisieren und/oder Pflegen von zahnärztlichen Instrumenten;
- Figur 4: den zeitlichen Ablauf der Wiederaufbereitung von vier Instrumenten bei dem Ausführungsbeispiel von Fig. 3;
- Figur 5: ein drittes Ausführungsbeispiel eines erfindungsgemäßen Geräts zum Desinfizieren, Sterilisieren und/oder Pflegen von zahnärztlichen Instrumenten;
- Figur 6: den zeitlichen Ablauf der Wiederaufbereitung von vier Instrumenten bei dem Ausführungsbeispiel von Fig. 5;
- Figur 7: ein viertes Ausführungsbeispiel eines erfindungsgemäßen Geräts zum Desinfizieren, Sterilisieren und/oder Pflegen von zahnärztlichen Instrumenten;
- Figuren 8a bis 8c: Ansichten eines Sprüharms zum Zuführen von Heißdampf;
- Figuren 9 und 10: Ansichten einer bevorzugten Ringanordnung von Düsen zum Aufbringen von Heißdampf; und
- Figur 11: ein fünftes Ausführungsbeispiel eines erfindungsgemäßen Geräts zum Desinfizieren, Sterilisieren und/oder Pflegen von zahnärztlichen Instrumenten.

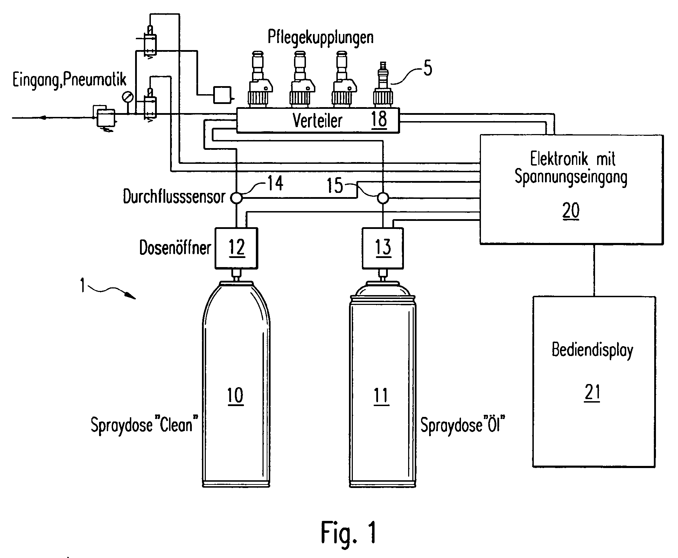

Figur 1 zeigt zunächst schematisch die Ausgestaltung einer ersten Ausführungsbeispiels eines erfindungsgemäßen Geräts zum Desinfizieren, Sterilisieren und/oder Pflegen von ärztlichen, insbesondere zahnärztlichen Instrumenten, wobei das Gerät nachfolgend allgemein mit dem Bezugszeichen 1 versehen ist.

Zentrales Element des erfindungsgemäßen Pflegegeräts 1 ist eine Pflege- und Reinigungskammer, in der während des Prozessablaufs die zu reinigenden bzw. pflegenden Instrumente angeordnet sind. Die Anordnung der Instrumente erfolgt hierbei mit Hilfe eines Instrumententrägers, auf dem mehrere Steckplätze bzw. Kupplungen 5 angeordnet sind. Vorzugsweise sind unterschiedliche Kupplungen 5 vorgesehen, sodass Instrumente mit Kupplungssystemen verschiedener Hersteller aufbereitet werden können.

Die Reinigung bzw. Pflege der Instrumente erfolgt bei dieser ersten Variante zum einen durch ein Reinigungsmittel sowie zum anderen durch ein Ölpflegemittel. Beide Medien sind in entsprechenden Vorratsbehältern 10 und 11 gelagert, welche in Form von Spraydosen zur Verfügung stehen. Sie sind auswechselbar an Anschlussmitteln des Geräts 1 angeordnet und können dementsprechend bei Bedarf gewechselt werden. Die Anschlussmittel umfassen hierbei insbesondere speziell ausgestaltete Mittel 12 und 13, über welche wahlweise die an der Oberseite der Spraydosen 10, 11 befindlichen Ventile geöffnet werden können (sog. Dosenöffner), um das entsprechende Mittel zu entnehmen. Eine exakte Dosierung des jeweiligen Mediums wird durch Durchflusssensoren 14 und 15 unterstützt, welche mit einer elektronischen Steuereinheit 20 verbunden sind. Diese steuert auch eine an den Pflegekupplungen 5 befindliche Verteilereinrichtung 18 an, über welche das entsprechende Medium dann gemäß dem vorgesehenen Verfahrensablauf an die einzelnen Kupplungen 5 weitergeleitet wird. Über ein Bediendisplay 21, welches beispielsweise als Touchscreen ausgebildet sein kann, kann dann der Aufbereitungsprozess gestartet werden. Es besteht ferner auch die Möglichkeit, über das Display 21 manuell die Anzahl und Positionen der aufzubereitenden Instrumente einzugeben. Alternativ wäre allerdings auch denkbar, diese Informationen automatisch zu erfassen.

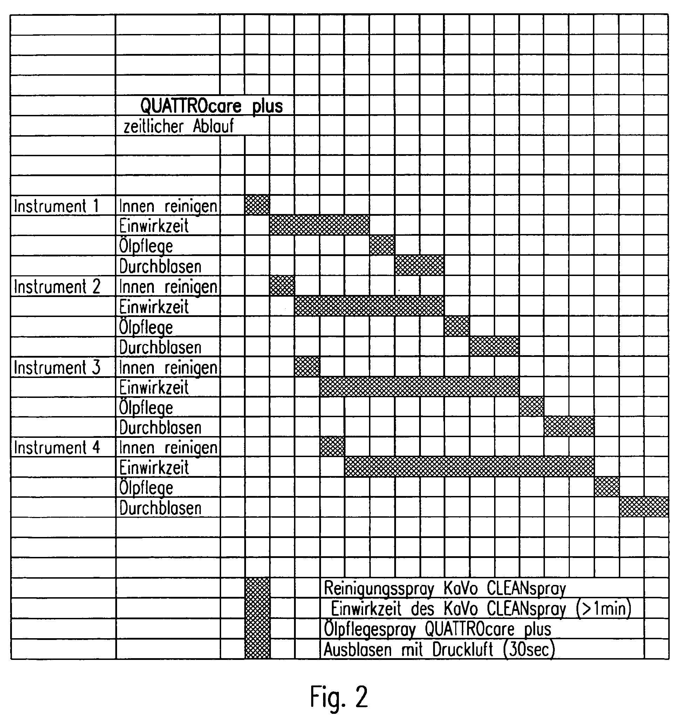

Entsprechend der schematischen Darstellung von Figur 2 ergibt sich dann - bei Belegung aller Steckplätze 5 - folgender zeitlicher Ablauf:

Die zur Aufbereitung vorbereiteten dentalen Instrumente (beispielsweise Hand-und Winkelstücke oder Turbinen) werden von der Bedienperson auf die Pflegekupplungen 5 des Geräts gesteckt. Der Aufbereitungsvorgang wird dann gestartet.

Hierbei wird zunächst ein Reinigungsmittel, welches sich in der ersten Dose 10 befindet, in Pulsen durch den Antriebskanal und die Spraykanäle der Instrumente gespült. Auf diesem Wege werden Proteine, Speichel und weitere organische Verschmutzungen aus dem Instrumenteninneren gespült. Nach dem Ablauf einer Einwirkzeit wird das Instrument mit einem Ölpflegemittel, welches in der Dose 11 bevorratet ist, innen behandelt. Hierbei wird vor allem der Abrieb, der durch mechanische Verunreinigungen gebildet ist, aus den Lagerstellen entfernt und mit frischem Öl versorgt. Um den Doseninhalt während der Aufbereitung zu überwachen sind die zuvor genannten Durchflusssensoren 14 und 15 den Spraydosen 10, 11 nachgeschaltet. Überschüssiges Öl und restliches Reinigungsmittel wird zum Abschluss des Verfahrens noch mit Druckluft aus den Sprayrohren und dem Antriebskanal der Instrumente ausgeblasen.

Dieser Aufbereitungszyklus erfolgt dabei nicht für alle Instrumente zeitgleich. Stattdessen beginnt die Innenreinigung für ein nachfolgendes Instrument dann, wenn für das vorhergehende Instrument die Einwirkzeit für das Reinigungsmittel beginnt. Dies hat zur Folge, dass die entsprechenden Medien aus den Vorratsbehältern zu einem bestimmten Zeitpunkt jeweils individuell einer Kupplung zugeführt werden müssen, was eine genauere und exakte Dosierung der Medien ermöglicht. Die Einwirkzeit für die Instrumente nimmt dabei zu, wie dies Figur 2 entnommen werden kann. Auf diesem Wege ist wiederum sichergestellt, dass auch die weiteren Medien (Öl und Druckluft) individuell den Instrumenten zugeführt werden.

Alternativ zu der zuvor beschriebenen Vorgehensweise wäre es auch möglich, die Spraydose mit dem Reinigungsmittel 10 durch einen Behälter zu ersetzen, welcher das Reinigungsmittel in flüssiger Form enthält. Dieses würde dann über eine Pumpe, zum Beispiel ein Plungerpumpe durch die entsprechenden Instrumente gedrückt. In gleicher Weise kann auch mit dem Öl verfahren werden, wobei dieses dann einem Luftstrom in geringer Menge zudosiert und von diesem zu den Lagerstellen des zu pflegenden Instruments befördert wird.

Bei einer anderen bevorzugten Alternative wird das Reinigungsmittel dosiert als Nebel zerstäubt in den jeweiligen Getriebe- und Spraykanal des Instruments eingebracht. Das Zerstäuben kann durch Tränken eines Sinterfilters erfolgen, der anschließend mit Druckluft durchblasen wird, wobei der sich hierbei bildende Nebel in das Instrument befördert wird. Während der Einwirkzeit kann mittels weiterer Druckluftstöße die Reinigungslösung bewegt werden. Nach Ablauf der Einwirkzeit wird dann mit Hilfe einer Pumpe, beispielsweise einer Magnetpumpe, Wasser gefördert und der Getriebe-und Spraykanal des Instruments gespült. Anschließend erfolgt das Ausblasen des Wassers mittels Druckluft. Der Ablauf kann bezüglich der Zeit, den Pulsen, der Menge der Reinigungsmittels und der Wiederholungen optimiert werden, wobei allerdings wiederum vorgesehen ist, dass die Zyklen für die einzelnen Instrumente versetzt gestartet werden, also jedes Instrument separat und dementsprechend gezielt gereinigt wird. Gleiches gilt auch für die anschließende Ölpflege, wobei wiederum Öl vorzugsweise als Nebel zerstäubt in den Getriebekanal eingebracht wird und die Dosierung des Öls beispielsweise über eine Zeitsteuerung erfolgt. Das Zerstäuben des Öls zum Bilden des Nebels kann in der oben genannten Weise erfolgen, auch die Ölschmierung erfolgt für jedes Instrument einzeln. Anschließend wird das überschüssige Öl aus dem Instrument entfernt und der Getriebe-und Spraykanal mit Druckluft gespült. Dies kann insbesondere auch pulsweise erfolgen, um das Verdrängen des überschüssigen Öls zu verbessern.

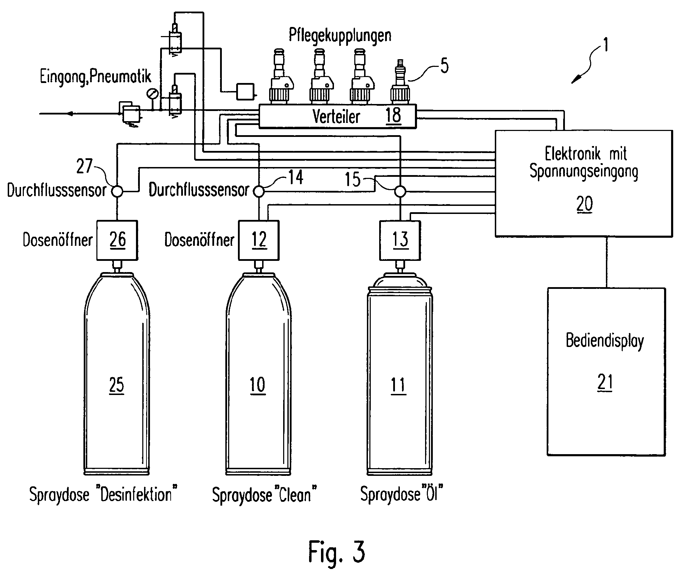

Figur 3 zeigt eine Weiterbildung des Geräts von Figur 1, wobei gleiche Elemente mit den gleichen Bezugszeichen versehen sind. Das Gerät 1 von Figur 3 unterscheidet sich dabei darin, dass hier zusätzlich eine Desinfektion der aufzubereitenden Instrumente erfolgen soll. Hierfür wird ein wiederum in einer Spraydose 25 bereitgestelltes Desinfektionsmittel genutzt. Der Spraydose 25 sind ebenso wie den beiden Dosen 10 und 11 Mittel zum Öffnen des Ventils 26 sowie ein Durchflusssensor 27 nachgeordnet.

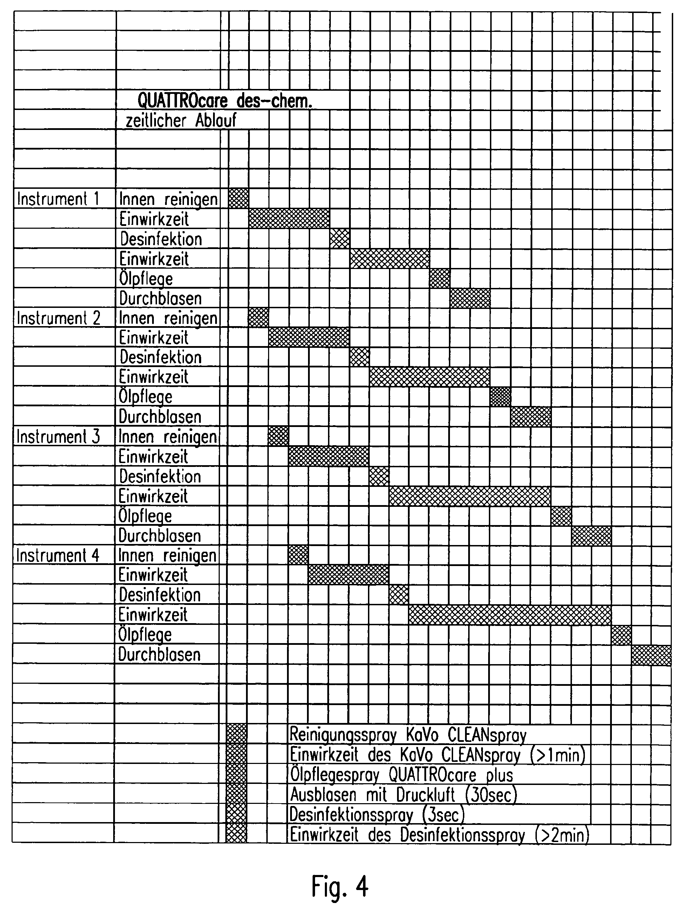

Der in Figur 4 dargestellte Verfahrensablauf entspricht dabei im Wesentlichen dem Ablauf von Figur 2, wobei nunmehr allerdings nach dem Einwirken des Reinigungsmittels die Desinfektion der Instrumente erfolgt, wobei hierzu vorzugsweise einmalig für drei Sekunden das entsprechende Desinfektionsmittel durch die Instrumente gesprüht wird. Hieran schließt sich eine Einwirkzeit von mindestens zwei Minuten Dauer an, welche - wie in Figur 4 dargestellt - für die einzelnen Instrumente jeweils zunimmt. Die Einwirkzeit für das Reinigungsmittel ist in diesem Fall für alle Instrumente gleich.

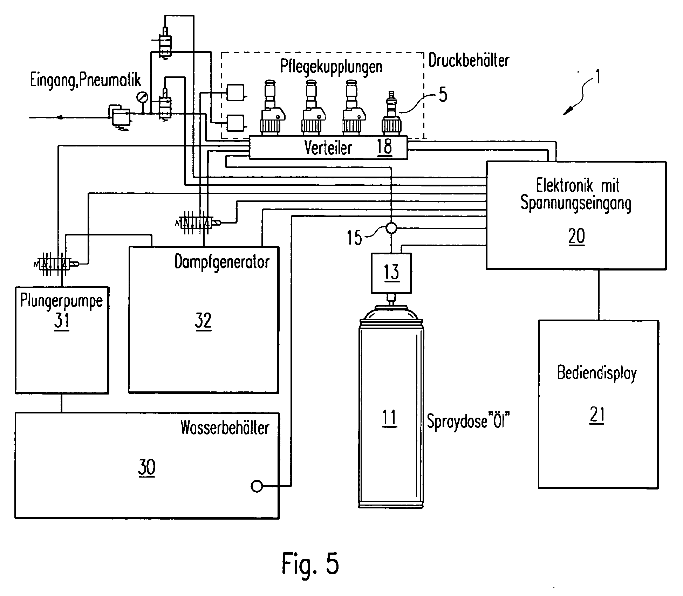

Bei der dritten Variante gemäß Figur 5 ist vorgesehen, dass zur Reinigung und Desinfektion der Instrumente Heißdampf genutzt wird. Dieser ist sehr energiereich und er überträgt aufgrund seiner physikalischen Eigenschaften auch Temperaturen sehr gut und schnell, was bei der vorliegenden Variante genutzt wird. Wiederum sind gleiche Elemente mit den gleichen Bezugszeichen versehen.

Das Gerät 1 weist dementsprechend zunächst einen Frischwasserbehälter 30 auf, aus dem mittels einer Pumpe 31 Wasser, vorzugsweise demineralisiert, in einen Dampfgenerator 32 gepumpt wird. Dort wird das Wasser erhitzt und in Heißdampf mit einer Temperatur von mehr als 120° Celsius umgewandelt.

Das aufzubereitende Instrument, welches auf einer der Kupplungen 5 angeordnet ist, wird dann nach einem Startimpuls circa 20 Sekunden lang mit Heißdampf durchströmt. Dabei werden von dem Dampf sowohl die Spraykanäle als auch der Getriebekanal durchströmt, um eine Innenreinigung durchzuführen. Bei diesem Vorgang erwärmt sich das Instrument auf etwa 98° Celsius. Anschließend erfolgt in gleicher Weise eine Außenreinigung des Instruments, indem dieses mit Heißdampf besprüht wird.

Der Effekt der Desinfektion beruht bekanntlicherweise auf Temperaturen um etwa 100° Celsius. Um diesen Desinfektionsvorgang effektiver zu gestalten, kann die Reinigungskammer des Geräts 1 abgedichtet werden, so dass ein Überdruck entsteht. Dies ermöglicht es, Temperaturen oberhalb von 100° Celsius zu erzielen. Die Desinfektionszeit kann in diesem Fall auf wenige Sekunden reduziert werden.

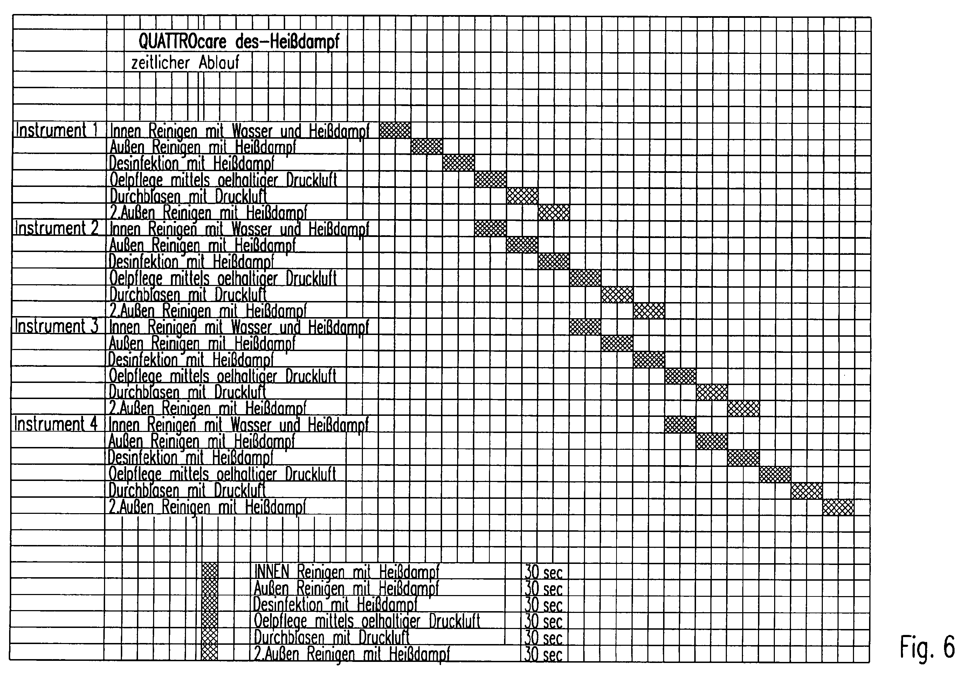

Im nächsten Schritt (siehe Figur 6) erfolgt dann die Ölpflege, wobei wie bei den zuvor beschriebenen Varianten wiederum das Ölpflegemittel aus der Spraydose 11 entnommen und durch die Instrumente gesprüht wird. Die Nutzung eines Sprays bringt den weiteren Vorteil mit sich, dass durch die Entspannung des Treibgases Kälte entsteht, welche das erwärmte Instrument abkühlt. Eventuell überschüssiges Öl kann dann über die gefilterte Druckluft aus dem Instrumenteninneren gedrückt werden. Abschließend erfolgt - vorzugsweise mittels einer nachfolgend noch näher beschriebenen beweglichen Ringdüse, welche über das Instrument geführt wird - eine weitere Außenreinigung. Hierbei wird insbesondere das Instrument an der Oberfläche von ausgetretenem Öl mittels Heißdampf gereinigt.

Wiederum erfolgen die Aufbereitungszyklen für die verschiedenen Instrumente versetzt, wobei der Zyklus für ein Instrument dann gestartet wird, wenn das vorherige Instrument mit Öl gepflegt wird.

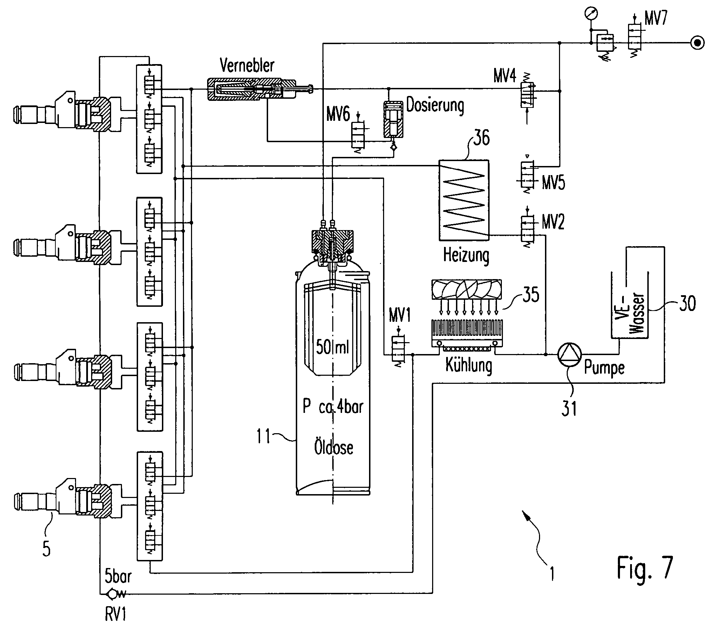

Eine Variante der zuvor beschriebenen Vorrichtung ist in Figur 7 dargestellt. Der Vorteil dieser Variante besteht in einer guten und schnellen Reinigung bei gleichzeitig desinfizierender Wirkung.

Bei dieser Vorrichtung erfolgt zunächst eine Innenreinigung der Instrumente mit kaltem Wasser um Blut und weitere Rückstände zu entfernen. Hierbei werden das Magnetventil MV1 und die Pumpe 31 aktiviert. Kaltes Wasser durchspült dann das Instrument. Durch ein Kühlaggregat 35 wird das Wasser abgekühlt um Blutreste möglichst gut anlösen zu können. Die Pumpe 31 ist vorzugsweise eine Magnetpumpe, die einen hohen pulsierenden Druck erzeugt. Das Kühlaggregat 35 kann beispielsweise in Form eines Peltierelements, eines Kompressors oder Wirbelrohrs ausgeführt sein. Die Schläuche bzw. Leitungen sollten möglichst stabil ausgeführt sein, um die Druckstöße gut weiterzuleiten. Nach einer Pause kann dieser Zyklus mehrfach wiederholt werden, um die Reinigungswirkung zu erhöhen.

An die Reinigung mit dem kalten Wasser schließt sich eine Innenreinigung mit Dampf an, um Ölverschmutzungen zu lösen und eine Innendesinfektion zu erzielen. Hierzu wird das Ventil MV2 geöffnet. Mittels der Pumpe 31 wird Wasser in den Heizkessel 36 eingespritzt und dieses in die Dampfphase überführt. Der Dampf durchströmt dann das Instrument, reinigt und desinfiziert dieses. Anschließend wird das Magnetventil MV4 geöffnet. Druckluft strömt in das Instrument und treibt Wasser aus und kühlt dieses gleichzeitig ab. Hierbei kann zur Pflege des Instruments gleichzeitig auch Öl aus dem Vorratsbehälter 11 in die Druckluft beigegeben werden.

Die verschiedenen Reinigungs- und Pflegeschritte für die Instrumente werden wiederum versetzt gestartet, um eine genauere Dosierung und damit Reinigung der Instrumente zu erzielen.

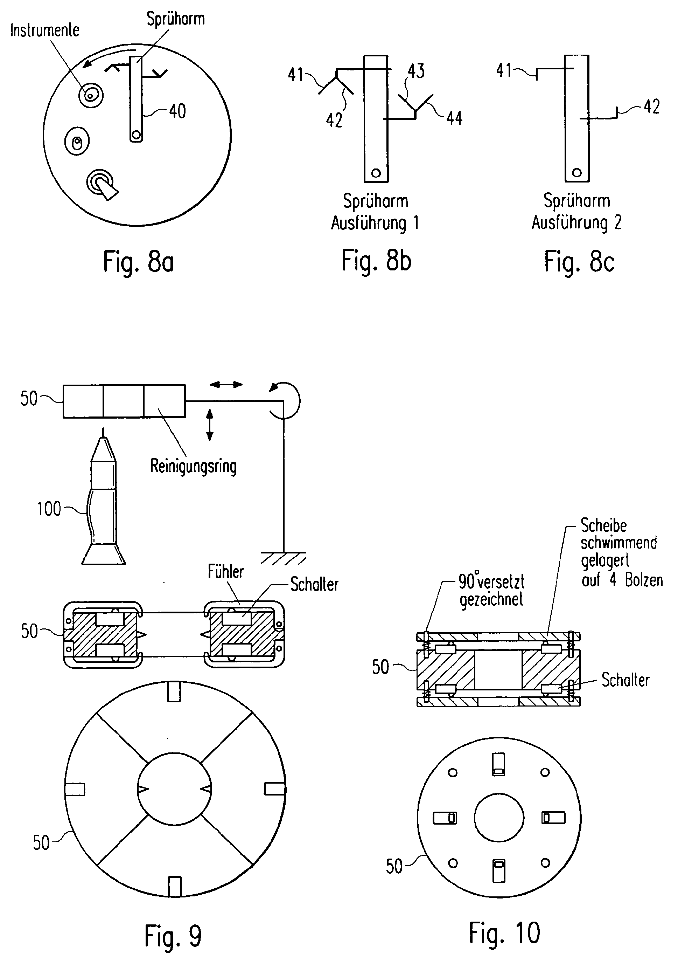

Insbesondere bei der Nutzung von Heißdampf zum Reinigen der Instrumente ist es erforderlich, das Reinigungsmittel, also den Heißdampf effektiv auf die Außenfläche der Instrumente zu richten. Dies kann beispielsweise durch einen speziell ausgestalteten Sprüharm erfolgen, der in den Figuren 8a bis 8b dargestellt ist. Die Besonderheit besteht dabei darin, dass der Sprüharm 40 sich dreht und in der Z-Achse bewegt werden kann. Er besitzt ferner mehrere Düsen 41 bis 44, aus denen der Dampf austritt. Beim Drehen des Arms 40 wird von der Düse 41 auf die weiteren Düsen 42, 43 und 44 umgeschaltet. Um über die ganze Höhe des Instruments hinweg reinigen zu können, werden entweder die einzelnen Düsen 41 bis 44entlang des Instruments bewegt oder es sind mehrere streifenförmig angeordnete Düsen vorhanden.

Die Figuren 8b und 8c zeigen verschiedene Möglichkeiten zur Anordnung der Düsen, wobei die Variante in Figur 8b gegenüber der Variante von Figur 8c den Vorteil mit sich bringt, dass der Dampfstrahl zwischen 0° und 45° auf das Instrument trifft. Im Gegensatz hierzu kann der Winkel bei der Variante von Figur 8c auch 180° betragen.

Die Figuren 9 und 10 zeigen schließlich eine weitere Möglichkeit, die Außenseite eines Instruments mittels Heißdampf zu reinigen. Wesentlicher Bestandteil dieser Anordnung ist jeweils ein Ring 50, der in allen drei Achsen bewegbar ist. Er besitzt mehrere taktile Segmente sowie Düsen an seiner Innenseite, aus denen der Dampf austritt. Die Düsen können beispielsweise auf einem Innenring drehbar gelagert sein, wobei der Rückstoß dann die Düsen rotieren lässt. Der Außenradius entspricht hierbei mindestens der Außenmittigkeit eines Winkelstücks.

Die Nutzung dieser Anordnung ist nunmehr wie folgt. Ein entsprechender Arm bewegt wie in Figur 9 schematisch dargestellt den Ring 50 über das Instrument 100. Der Ring 50 wird dann nachfolgend abgesenkt. Falls das Instrument 100 nicht zentrisch getroffen wird, löst ein Schalter 53 des Rings 50 aus. Hierdurch ist bekannt, in welche Richtung der Ring 50 zu bewegen ist. Dabei wird immer solange verfahren, bis alle Schalter des Rings 50 frei sind. Anschließend wird der Ring 50 weiter abgesenkt, bis wieder einer der Schalter auslöst. Während dieses Vorgangs wird kontinuierlich Heißdampf auf die Außenseite des Instruments 100 gesprüht.

Um anschließend den Ring 50 wieder zur Oberseite zu bewegen, befinden sich an der Oberseite des Rings in gleicher Weise Schalter bzw. Fühler. Alternativ hierzu wäre es auch denkbar, bei der Abwärtsbewegung die ermittelte Kontur zu speichern und diese in umgekehrter Richtung zur Aufwärtsbewegung abzufahren. Letztendlich kann durch die Nutzung dieses speziellen Rings 50 der Heißdampf effektiv auf die Instrumente aufgebracht werden, um die reinigende und desinfizierende Eigenschaft des Dampfs zu nutzen.

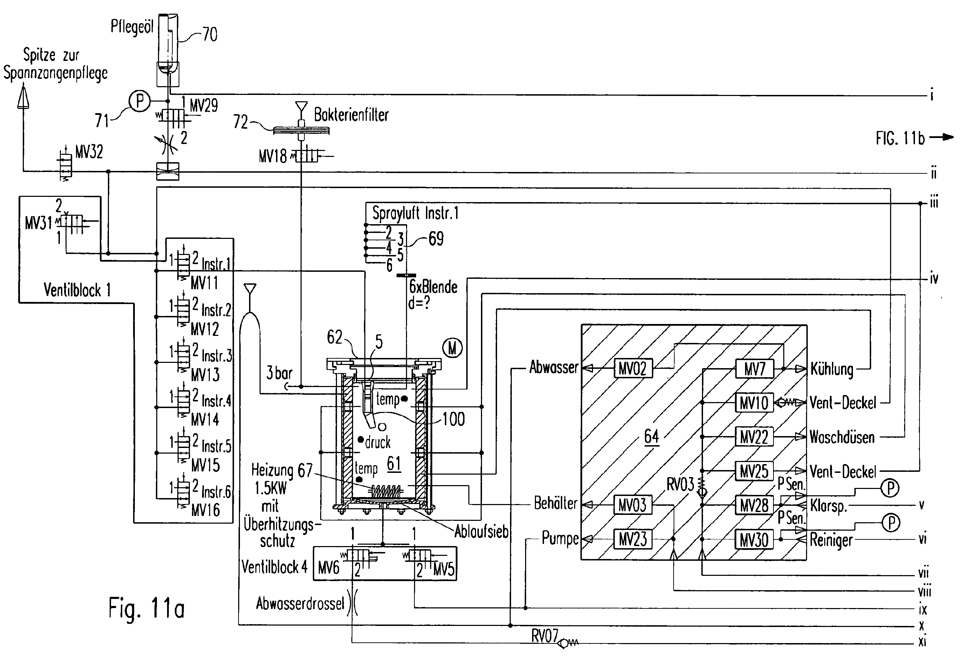

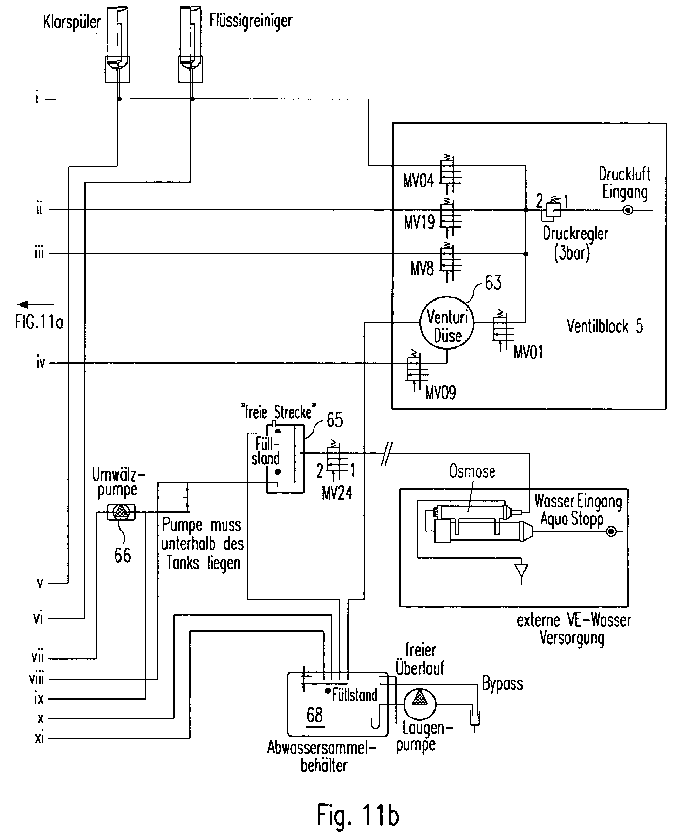

Anhand der Figuren 11a und 11b soll schließlich noch ein weiteres Ausführungsbeispiel eines erfindungsgemäßen Geräts zum Desinfizieren, Sterilisieren und/oder Pflegen von zahnärztlichen Instrumenten beschrieben werden. Die Figuren 11 a und 11b zeigen hierbei den schematischen Aufbau eines Geräts, bei dem einzelne Instrumente in sechs aufeinanderfolgenden Einzelabschnitten aufbereitet werden. Es handelt sich insbesondere um folgende Arbeitsschritte: Waschen, Ölpflege, Klarspülen, Sterilisieren, Trocknen und Kühlen. Wie nachfolgend erläutert wird kann dabei jeder Einzelschritt in weitere Schritte unterteilt sein. Die Vorgehensweise bei der Aufbereitung der verschiedenen Instrumente ist dabei wie folgt:

### a) Initialisierung:

Die Wiederaufbereitung der Instrumente beginnt zunächst mit der Initialisierung des Gerätes. Dabei werden die Füllstände in den verschiedenen Vorratsbehältern und Drücke überprüft und die in der Pflegekammer 61 vorhandenen Kupplungen 5 auf Belegung abgefragt. Dies geschieht, indem Überdruck in die Pflegekammer 61 geleitet wird und darauf ein Ventil (MV 11 bis MV16) geöffnet wird. Der Überdruck entweicht über die gegebenenfalls belegte Kupplung 5 über das entsprechende Ventil - z.B. MV 11 - und MV31 in die Atmosphäre. Über den Druckverlust in einer bestimmten Zeit wird festgestellt, ob die angesprochene Pflegekupplung 5, also die dem entsprechenden Ventil zugeordnete Pflegekupplung 5 belegt oder frei ist.

### b) Waschen

Die wiederaufzubereitenden Instrumente 100 werden auf den Träger 62, der zugleich den Deckel der Pflegekammer 61 bildet, aufgesteckt und dieser dann in / auf der Pflegekammer 61 platziert. Mittels einem motorischen Verschluss wird der Deckel fest mit der Pflegekammer 61 verbunden und diesem damit verschlossen. Anschließend wird mit Hilfe einer Venturidüse 63 in der Pflegekammer 61 ein Unterdruck erzeugt und in dem Ventilblock "Wasser" 64 das zur Kammer 61 führende Ventil MV03 geöffnet. Waschwasser fließt nun vom Behälter 65 in die Pflegekammer 61. Nach dem Befüllten schaltet das MV03 ab und die Umwälzpumpe 66 wird aktiviert. Das Waschwasser nimmt hierbei den Weg über die Ventile MV22, die Waschdüsen, das Ventil MV5 wieder zur Umwälzpumpe 66. Nach einer kalten Außenreinigung der Instrumente 100 erfolgt eine kalte Innenreinigung, wozu die Ventile MV10 und das entsprechende Ventil der dazugehörigen Pflegekupplung MV11 bis MV 16 sequenziell geöffnet werden. Das in der Pflegekammer 61 befindliche Waschwasser wird wieder über das MV5 zur Umwälzpumpe 66 geführt.

Nach der Kaltwäsche wird über das Ventil MV30 dem Waschwasser Flüssigreiniger hinzugefügt und die Heizung 67 zur Erwärmung des Waschwassers eingeschaltet. Diese Warmwäsche schließt mit dem Ausschalten der Heizung 67, der Umwälzpumpe und der Ventile MV 22, MV 10 und MV 5 ab. Das Ventil MV6 wird geöffnet und das Waschwasser kann in den Abwassersammelbehälter 68 eingeleitet werden. Der Vorgang der Entleerung kann durch Zufuhr von Druckluft über das Ventil MV 8 und die Verteilereinrichtung 69 in die Pflegekammer 61 unterstützt werden. Ein Ablaufsieb am Boden der Pflegekammer 61 verhindert das Eindringen von gröberen Verschmutzungen in das Rohrsystem heraus.

Programmtechnisch kann alternativ zu der beschriebenen Vorgehensweise auch zuerst die Innenreinigung und darauf die Außenreinigung erfolgen.

### c) Pflege

Eine Öl-Pflege der Instrumente 100 erfolgt sequentiell. Hierdurch ist wiederum sichergestellt, dass jedes zu pflegende Instrument 100 die richtige Ölmenge erhält. Dabei wird der vorzugsweise als Zweikammerdose ausgebildet Öl-Vorratsbehälter 11 mit Druckluft beaufschlagt, was durch Öffnen des Ventils MV04 erfolgt. Der Innenbeutel der Öldose 11, der das Pflegeöl enthält, steht nun unter einem entsprechenden Außendruck. Durch Öffnen des Ventils MV29 fließt Öl über die Venturidüse in einen Luftstrom. Die Ölmenge ist dabei abhängig von der Öffnungszeit des Ventils MV29 und dem freien Querschnitt der darauffolgenden Drossel. Der Luftstrom, ausgelöst durch das Öffnen von Ventil MV 19 trägt das nun vernebelte Öl über ein mit dem zu pflegenden Instrument 100 verbundenes Magnetventil (MV 11 bis MV16). Befindet sich im Innenbeutel des Dose 11 kein Öl mehr, so herrscht dort auch kein Druck. Diesen Druckabfall registriert der Drucksensor 71 und meldet ggf. dass die Öldose leer ist.

### d) Klarspülen:

Der sich anschließende Schritt des Klarspülens wird benötigt um eventuell an der Oberfläche der gepflegten Instrumente 100 befindliches Öl abzuspülen und um zu verhindern, dass sich Wasserflecken auf den Instrumenten bilden.

Dazu wird die Pflegekammer 61, wie oben bereits beschrieben, mit Wasser gefüllt. Diesem wird über das Ventil MV 28 Klarspüler zudosiert. Um die Wirkung des Klarspülers zu nutzen wird das Wasser über die Heizung 67 auf ca. 70°C erhitzt um dann über die Umwälzpumpe und die Waschdüsen die Instrumente klar zu spülen. Nach erfolgtem Vorgang wird die Flüssigkeit, wie ebenfalls bereits beschrieben, aus der Pflegeklammer 61 in den Abwassersammelbehälter 68 geleitett.

### e) Sterilisieren:

Es ist möglich, eine Sterilisation der Klassen "B" oder "S" bzw. auch nur eine Desinfektion durchzuführen. Das aufwändigste Verfahren der Klasse "B" soll beschrieben werden.

Die zur Klasse "B" notwendige zwei- oder dreifache Evakuierung der Pflegekammer 61 wird beim vorliegenden Ausführungsbeispiel über die Vakuumdüse erzielt. Hierzu sind alle Ventile, die zur Pflegekammer 61 führen, geschlossen. Lediglich das Ventil MV9 ist geöffnet. Durch anschließendes Öffnen des Ventils MV01 wird dann über die Venturidüse 63 in der Pflegekammer das nötige Vakuum erzeugt. Die Abluft der Venturidüse 63 wird in den Abwassersammelbehälter 68 geleitet.

Der zur Sterilisation notwendige Wasserdampf wird durch erhitzen der Bodenplatte der Pflegekammer 61 und Einspritzen von Wasser auf diese Bodenplatte erzeugt. Nach Erreichen der Parameter, die für eine Sterilisation notwendig sind (Druck, Temperatur) wird diese Einstellung über die Dauer der Sterilisationszeit konstant gehalten.

Anschließend wird der Druck über das Ventil MV6 abgelassen (in den Abwassersammelbehälter 68) und der Druckausgleich über das Ventil MV 18 und den davor angeordneten Bakterienfilter 72 vorgenommen.

Ein Sterilisationsverfahren der Klasse "S" oder ein Desinfektionsverfahren läuft ähnlich ab, wobei allerdings bei der Klasse "B" kein Vakuum erzeugt wird und beim Desinfektionsverfahren die Temperaturen niedriger sind.

### f) Trocknen und Kühlen:

Nach jeder Sterilisation sind die Instrumente 100 durch den Wasserdampf innen wie außen nass. Um die Instrumente 100 zu trocknen wird beim vorliegenden Gerät ein Kondensationsverfahren angewendet, wozu der Raum zwischen Innenkammer und Außenkammer der Pflegekammer 61 mit Kaltwasser geflutet wird. Dazu wird von der Pumpe 66 über das geöffnete Ventil MV 23 Kaltwasser über das Ventil MV 7 in den Raum zwischen den Wandungen gefördert. Hierdurch kühlt sich die Pflegekammer 61 ab und an der nun kalten Innenwand kondensiert jeglicher Wasserdampf, der sich in der Pflegekammer befindet. Zur Unterstützung der Trocknung und Kühlung der Instrumente 100 kann gefilterte Druckluft zur Luftbewegung in die Pflegekammer 61 eingeleitet werden. Ist der Raum zwischen den Behältern mit Wasser gefüllt, das nun erwärmt ist, wird dieses warme Wasser über eine Wasserleitung dem Abwassersammelbehälter zugeführt. Nach Erreichen der Trocknung und einer vertretbaren Temperatur der Instrumente 100 ist der Wiederaufbereitungsvorgang abgeschlossen.

Insgesamt wird somit durch die vorliegende Erfindung die Möglichkeit geschaffen, zahnärztliche Instrumente in effektiver und komfortabler Weise wiederaufzubereiten.

## Patentansprüche

1. Gerät (1) zum Desinfizieren, Sterilisieren und/oder Pflegen von ärztlichen, insbesondere zahnärztlichen Instrumenten (100), aufweisend
• eine Pflege- und Reinigungskammer zur Aufnahme von aufzubereitenden Instrumenten sowie
• Mitteln zum Zuführen unterschiedlicher Reinigungs- oder Pflegemedien, um die Instrumente in aufeinanderfolgenden Schritten zu Reinigen, zu Desinfizieren bzw. Sterilisieren und/oder zu Pflegen,
wobei die Aufbereitungszyklen für die verschiedenen Instrumente zeitlich versetzt starten,
**dadurch gekennzeichnet,**
**dass** das Gerät eine elektronische Steuereinheit (20) aufweist, die mit Durchflusssensoren (14, 15) verbunden ist, wobei die Steuereinheit (20) dazu ausgebildet ist, die Aufbereitungszyklen zeitlich überlagert ablaufen zu lassen.

2. Gerät nach Anspruch 1,
**dadurch gekennzeichnet,**
**dass** eine Reinigung und Desinfektion der Instrumente durch Heißdampf erfolgt.

3. Gerät nach Anspruch 2,
**dadurch gekennzeichnet,**
**dass** ein Aufbereitungszyklus für ein Instrument jeweils aus folgenden aufeinanderfolgenden Schritten besteht:
• Innenreinigung mittels Heißdampf;
• Außenreinigung mittels Heißdampf;
• Desinfektion mittels Heißdampf;
• Ölpflege mittels ölhaltiger Druckluft;
• Durchblasen mittels Druckluft; und
• weitere Außenreinigung mittels Heißdampf.

4. Gerät nach Anspruch 3,
**dadurch gekennzeichnet,**
**dass** ein Aufbereitungszyklus für ein Instrument gestartet wird, wenn das vorherige Instrument mittels ölhaltiger Druckluft gepflegt wird.

5. Gerät nach einem der Ansprüche 1 bis 4,
**dadurch gekennzeichnet,**
**dass** dieses zum Aufbringen des Heißdampfs auf die Außenseite der Instrumente (100) ein rotierendes Element, insbesondere einen Arm (40) oder einen Ring (50), mit daran befindlichen Düsen aufweist.

6. Gerät nach Anspruch 1,
**dadurch gekennzeichnet,**
**dass** eine Desinfektion der Instrumente durch die Zuführung und das Einwirken lassen eines Desinfektionsmittels, insbesondere eines Desinfektionssprays erfolgt,
wobei die Einwirkzeit für nachfolgende Instrumente zunimmt.

7. Gerät nach Anspruch 6,
**dadurch gekennzeichnet,**
**dass** ein Aufbereitungszyklus für ein Instrument jeweils aus folgenden aufeinanderfolgenden Schritten besteht:
• Innenreinigung durch Zuführen und Einwirken lassen eines Reinigungsmittels;
• Desinfektion durch Zuführen und Einwirken lassen eines Desinfektionsmittels;
• Ölpflege durch Zuführen ein Ölpflegemittels; und
• Durchblasen des Instruments.

8. Gerät nach Anspruch 1,
**dadurch gekennzeichnet,**
**dass** eine Innenreinigung der Instrumente durch die Zuführung und das Einwirken lassen eines Reinigungsmittels, insbesondere eines Reinigungssprays erfolgt,
wobei die Einwirkzeit für nachfolgende Instrumente zunimmt.

9. Gerät nach Anspruch 8,
**dadurch gekennzeichnet,**
**dass** ein Aufbereitungszyklus für ein Instrument jeweils aus folgenden aufeinanderfolgenden Schritten besteht:
• Innenreinigung durch Zuführen und Einwirken lassen eines Reinigungsmittels;
• Ölpflege durch Zuführen ein Ölpflegemittels; und
• Durchblasen des Instruments mittels Druckluft.

10. Gerät nach Anspruch 7 oder Anspruch 9,
**dadurch gekennzeichnet,**
**dass** ein Aufbereitungszyklus für ein Instrument gestartet wird, wenn die Einwirkzeit für das Reinigungsmittel für das vorherige Instrument beginnt.

## Claims

1. Device (1) for disinfecting, sterilizing and/or maintaining medical, especially dental, instruments (100), said device (1) having
• a maintenance and cleaning chamber for receiving instruments that are to be conditioned, and
• means for supplying different cleaning or maintenance media for cleaning, disinfecting or sterilizing and/or maintaining the instruments in successive steps,
wherein the start times for the conditioning cycles for the various instruments are staggered,
**characterized in that**
the device has an electronic control unit (20) which is connected to flow sensors (14, 15), wherein the control unit (20) is designed to allow the conditioning cycles to run in a manner superposed in time.

2. Device according to Claim 1, **characterized in that** cleaning and disinfection of the instruments is carried out using superheated steam.

3. Device according to Claim 2, **characterized in that** a conditioning cycle for an instrument is in each case composed of the following successive steps:
• internal cleaning by superheated steam;
• external cleaning by superheated steam;
• disinfection by superheated steam;
• oil maintenance by oil-containing compressed air;
• blowing compressed air through; and
• further external cleaning by superheated steam.

4. Device according to Claim 3, **characterized in that** a conditioning cycle for an instrument is started when the preceding instrument is being maintained by oil-containing compressed air.

5. Device according to one of Claims 1 to 4, **characterized in that**, in order to apply the superheated steam to the outside of the instruments (100), the device has a rotating element, in particular an arm (40) or a ring (50), with nozzles located thereon.

6. Device according to Claim 1, **characterized in that** disinfection of the instruments is carried out by supplying a disinfectant, in particular a disinfectant spray, and allowing same to act on the instruments, wherein the period of action increases for subsequent instruments.

7. Device according to Claim 6, **characterized in that** a conditioning cycle for an instrument is in each case composed of the following successive steps:
• internal cleaning by supplying a cleaning agent and allowing the cleaning agent to act;
• disinfection by supplying a disinfectant and allowing the disinfectant to act;
• oil maintenance by supplying an oil maintenance agent; and
• blowing air through the instrument.

8. Device according to Claim 1, **characterized in that** internal cleaning of the instruments is carried out by supplying a cleaning agent, in particular a cleaning spray, and allowing same to act on the instruments, wherein the period of action increases for subsequent instruments.

9. Device according to Claim 8, **characterized in that** a conditioning cycle for an instrument is in each case composed of the following successive steps:
• internal cleaning by supplying a cleaning agent and allowing the cleaning agent to act;
• oil maintenance by supplying an oil maintenance agent; and
• blowing compressed air through the instrument.

10. Device according to Claim 7 or Claim 9, **characterized in that** a conditioning cycle for an instrument is started when the period of action for the cleaning agent for the preceding instrument begins.

## Revendications

1. Appareil (1) pour la désinfection, la stérilisation et/ou le traitement d'instruments médicaux, en particulier dentaires (100), présentant
- une chambre de traitement et/ou de nettoyage destinée à recevoir des instruments à préparer et
- des moyens pour acheminer différents agents de nettoyage ou de traitement pour nettoyer les instruments par étapes successives, à des fins de désinfection ou de stérilisation et/ou de traitement,
les cycles de préparation pour les différents instruments commençant de manière décalée dans le temps,
**caractérisé en ce que**
l'appareil présente une unité de commande électronique (20) qui est connectée à des capteurs de débit (14, 15), l'unité de commande (20) étant réalisée pour laisser se dérouler les cycles de préparation de manière superposée dans le temps.

2. Appareil selon la revendication 1,
**caractérisé en ce que**
un nettoyage et une désinfection des instruments sont effectués avec de la vapeur chaude.

3. Appareil selon la revendication 2,
**caractérisé en ce que**
un cycle de préparation pour un instrument se compose à chaque fois des étapes successives suivantes :
- nettoyage intérieur au moyen de vapeur chaude ;
- nettoyage extérieur au moyen de vapeur chaude ;
- désinfection au moyen de vapeur chaude ;
- traitement à l'huile au moyen d'air comprimé contenant de l'huile
- soufflage avec de l'air comprimé ; et
- nettoyage extérieur supplémentaire avec de la vapeur chaude.

4. Appareil selon la revendication 3,
**caractérisé en ce que**
un cycle de préparation pour un instrument est commencé lorsque l'instrument précédent est traité avec de l'air comprimé contenant de l'huile.

5. Appareil selon l'une quelconque des revendications 1 à 4,
**caractérisé en ce que**
celui-ci présente pour appliquer la vapeur chaude sur le côté extérieur des instruments (100) un élément rotatif, en particulier un bras (40) ou une bague (50) avec des buses situées sur celui-ci.

6. Appareil selon la revendication 1,
**caractérisé en ce que**
une désinfection des instruments peut être effectuée par alimentation et action d'un agent désinfectant, en particulier un spray désinfectant, le temps d'action augmentant pour les instruments suivants.

7. Appareil selon la revendication 6,
**caractérisé en ce que**
un cycle de préparation pour un instrument se compose à chaque fois des étapes successives suivantes :
- nettoyage intérieur par acheminement et action d'un agent de nettoyage ;
- désinfection par acheminement et action d'un agent désinfectant ;
- traitement à l'huile par acheminement d'un agent de traitement à l'huile ; et
- soufflage de l'instrument.

8. Appareil selon la revendication 1,
**caractérisé en ce que**
un nettoyage interne des instruments s'effectue par l'acheminement et l'action d'un agent de nettoyage, en particulier d'un spray de nettoyage, le temps d'action augmentant pour les instruments suivants.

9. Appareil selon la revendication 8,
**caractérisé en ce que**
un cycle de préparation pour un instrument se compose à chaque fois des étapes successives suivantes :
- nettoyage intérieur par acheminement et action d'un agent de nettoyage ;
- traitement à l'huile par acheminement d'un agent de traitement à l'huile ; et
- soufflage de l'instrument au moyen d'air sous pression.

10. Appareil selon la revendication 7 ou la revendication 9,
**caractérisé en ce que**
un cycle de préparation pour un instrument est commencé lorsque le temps d'action pour l'agent de nettoyage pour l'instrument précédent commence.
